# EUROPÄISCHE PATENTSCHRIFT

(11) **EP 0 813 518 B1**
(45) Veröffentlichungstag und Bekanntmachung des Hinweises auf die Patenterteilung: **23.12.1998**
(21) Anmeldenummer: 96905802.3
(22) Anmeldetag: 27.02.1996
(51) Int. Cl.: C07C 255/30, C07D 241/06, C07C 253/08

(54) **VERFAHREN ZUR HERSTELLUNG VON ALPHA,BETA-DIAMINOACRYLNITRILEN**
PROCESS FOR PREPARING ALPHA,BETA-DIAMINOACRYLNITRILES
PROCEDE DE PREPARATION D'ALPHA,BETA-DIAMINOACRYLONITRILES

(30) Priorität: 03.03.1995 DE 19507463
(43) Veröffentlichungstag der Anmeldung: 29.12.1997
(73) Patentinhaber: BASF AKTIENGESELLSCHAFT, 67056 Ludwigshafen (DE)
(72) Erfinder: HENNINGSEN, Michael, D-67227 Frankenthal (DE); KANDZIA, Christof, D-67157 Wachenheim (DE); DOYE, Sven, D-67067 Ludwigshafen (DE)
(86) Internationale Anmeldenummer: EP9600789
(87) Internationale Veröffentlichungsnummer: WO9627582

(56) Entgegenhaltungen:
- EP-A- 0 175 364

## Beschreibung

Die vorliegende Erfindung betrifft ein verbessertes Verfahren zur Herstellung von α,β-Diaminoacrylnitrilen der allgemeinen Formel I

R³R²NC(R¹)=C(NR²R³)CN I

in welcher
R¹, R² und R³ gleich oder verschieden und voneinander unabhängig Wasserstoff oder ein Alkyl-, Cycloalkyl-, Aralkyl-, Aryl- oder ein heterocyclischer Rest ist, wobei die organischen Reste R² auch unter Ausbildung eines 6- oder 7-gliedrigen Ringes oder R² und R³ unter Ausbildung eines 5- oder 6-gliedrigen Ringes miteinander verbunden sein können.
α,β-Diaminoacrylnitrile sind wertvolle Zwischenprodukte z.B. für die Herstellung von Pharmazeutika wie dies in DE-A-44 46 025.2 beschrieben ist.

Aus EP 0 175 364 ist bereits ein vorteilhaftes Verfahren zur Herstellung von α,β-Diaminoacrylnitrilen der allgemeinen Formel

R³R²NC (R¹)=C(NR²R³)CN

bekannt, in welcher
R¹ für Wasserstoff, aliphatische Reste mit 1 bis 30 Kohlenstoffatomen, cycloaliphatische Reste mit 3 bis 8 Ringgliedern oder für aromatische, heteroaromatische oder araliphatische Reste mit 7 bis 12 Kohlenstoffatomen steht, wobei diese Reste auch substituiert sein können;
R² und R³ gleich oder verschieden sind und Wasserstoff oder einen aliphatischen Rest mit 1 bis 15 Kohlenstoffatomen, einen cycloaliphatischen Rest mit 3 bis 8 Ringgliedern oder einen aromatischen, heterocyclischen oder araliphatischen Rest mit 7 bis 12 Kohlenstoffatomen bedeuten und wobei die Reste R² und R³ auch gemeinsam zu einem 5- bis 6-gliedrigen Ring verbunden sein können, der noch ein weiteres Heteroatom tragen kann, gemäß dem man die Komponente A) mit den Komponenten B), C) und D), das sind
   A) eine α-Dicarbonylverbindung der allgemeinen Formel R¹COCHO, in der R¹ die o.a. Bedeutung hat oder ein reaktionsfähiges Derivat dieser Carbonylverbindung;
   B) ein Salz der schwefligen Säure oder Schwefeldioxid oder ein Schwefeldioxid lieferndes Reagenz;
   C) ein Amin der allgemeinen Formel HNR²R³, oder ein aliphatisches Diamin der allgemeinen Formel R³HN-(CH₂)ₙ-NHR³, in dem die Alkylenkette noch einen Substituenten R¹ tragen kann, wobei R¹ und R³ die o.a. Bedeutung haben und n die Zahlen 2 oder 3 bedeutet, oder Derivate, die unter den Reaktionsbedingungen in das Amin bzw. Diamin übergehen;
   D) flüssige oder gasförmige Blausäure oder ein Blausäure lieferndes Reagenz in beliebiger Reihenfolge umsetzt.

Für die Kombination A) + B) + C) + D) ergibt sich folgendes Reaktionsschema:

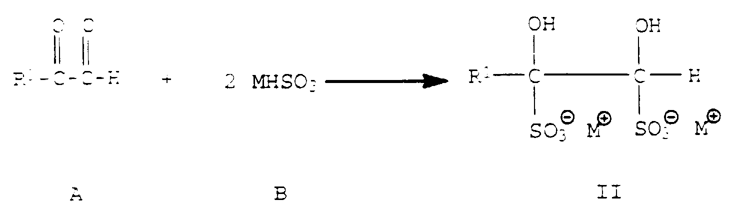

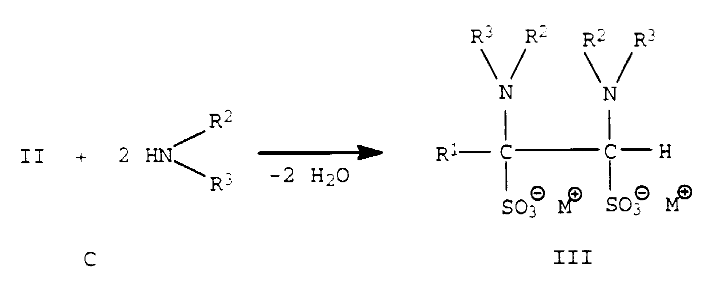

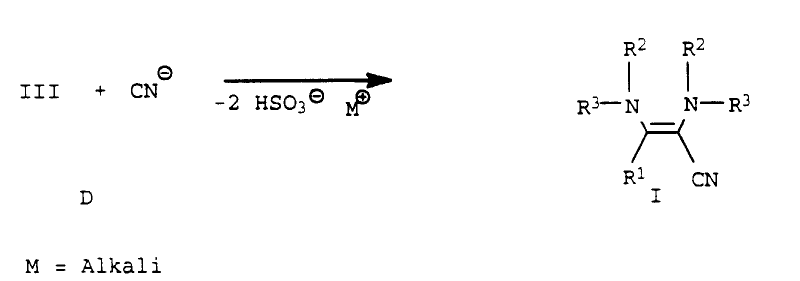

Die Reihenfolge des Zusammengebens der Komponenten A) - D) ist beliebig; man kann auch 2 oder mehr Komponenten gleichzeitig vereinigen oder aber das Gemisch von 2 oder mehr Komponenten mit den übrigen in beliebiger Weise umsetzen. Die Synthese von I läßt sich im "Eintopfverfahren" durchführen, man kann aber auch jede Zwischenstufe isolieren und weiter mit dem(n) noch fehlenden Reaktionspartner(n) zu I umsetzen.

Nach der Lehre der EP 0 175 364 werden als Komponente B vor allem Salze der schwefligen Säure, insbesondere Alkalisalze vorzugsweise Alkalihydrogensulfit in Betracht gezogen. Als Alkalihydrogensulfit wird in allen Beispielen, in denen ein Salz der schwefligen Säure verwendet wird, ausschließlich Natriumhydrogensulfit eingesetzt.

Die Aufarbeitung des Reaktionsgemisches erfolgt gemäß EP 0 175 364 S. 4, 1. Absatz, insbesondere durch Extrahieren, um das Reaktionsprodukt von dem mitausfallenden Natriumsulfit/hydrogensulfit abzutrennen. Dies gilt im besonderen Maße, wenn zur Ausbeutesteigerung zusätzlich Natronlauge zugegeben wird.

Die Verwendung von organischen Lösungsmitteln ist jedoch sowohl aus Sicherheits-, Kosten- als auch Umweltschutzgründen unerwünscht.

Es bestand daher die Aufgabe, das genannte Verfahren so zu verbessern, daß eine Abtrennung des Reaktionsproduktes ohne Verwendung von Lösungsmitteln möglich ist.

Diese Aufgabe wurde erfindungsgemäß mit einem verbesserten Verfahren zur Herstellung von α,β-Diaminoacrylnitrilen der allgemeinen Formel I gelöst

R³R²NC (R¹)=C(NR²R³)CN I,

in welcher
R¹ für Wasserstoff, aliphatische Reste mit 1 bis 30 Kohlenstoffatomen, cycloaliphatische Reste mit 3 bis 8 Ringgliedern oder für aromatische, heteroaromatische oder araliphatische Reste mit 7 bis 12 Kohlenstoffatomen steht, wobei diese Reste auch substituiert sein können;
R² und R³ gleich oder verschieden sind und Wasserstoff oder einen aliphatischen Rest mit 1 bis 15 Kohlenstoffatomen, einen cycloaliphatischen Rest mit 3 bis 8 Ringgliedern oder einen aromatischen, heterocyclischen oder araliphatischen Rest mit 7 bis 12 Kohlenstoffatomen bedeuten und wobei die Reste R² und R³ auch gemeinsam zu einem 5- bis 6-gliedrigen Ring verbunden sein können, der noch ein weiteres Heteroatom tragen kann, oder in der die Reste R² gemeinsam eine Alkylenkette mit 2 bis 3 Kohlenstoffatomen bilden, die noch einen Substituienten R¹ tragen kann durch Umsetzung der Komponente A) mit den Komponente B), C) und D), das sind
   A) eine α-Dicarbonylverbindung der allgemeinen Formel R¹COCHO, in der R¹ die o.a. Bedeutung hat oder ein reaktionsfähiges Derivat dieser Carbonylverbindung;
   B) ein Salz der schwefligen Säure oder Schwefeldioxid oder ein Schwefeldioxid lieferndes Reagenz;
   C) ein Amin der allgemeinen Formel HNR²R³, oder ein aliphatisches Diamin der allgemeinen Formel R³HN-(CH₂)ₙ-NHR³, in dem die Alkylenkette noch einen Substituenten R¹ tragen kann, wobei R¹ und R³ die o.a. Bedeutung haben und n die Zahlen 2 oder 3 bedeutet, oder Derivate, die unter den Reaktionsbedingungen in das Amin bzw. Diamin übergehen;
   D) flüssige oder gasförmige Blausäure oder ein Blausäure lieferndes Reagenz
      in beliebiger Reihenfolge, indem man die Umsetzung, bezogen auf ein Mol der Komponente A, mit mindestens 3,5 Äquivalenzen Alkaliverbindungen durchführt, wovon der überwiegende Teil aus Kaliumverbindungen besteht und wobei mindestens ein Äquivalent Alkali in Form von freier Alkalilauge zugesetzt wird, mit der Maßgabe, daß man den Anteil an Kaliumäquivalenten mindestens so hoch wählt, daß am Ende der Reaktion alle anorganischen Salze praktisch vollständig in Lösung bleiben.
      In der Regel sind dazu mindestens 2,5 Äquivalente Kalium erforderlich.

Nach einer besonders bevorzugten Ausführungsform führt man die Umsetzung ausschließlich mit Kaliumverbindungen durch, wobei die Gesamtmenge des Kaliums, bezogen auf die Komponente A, mindestens 4 Äquivalente beträgt mit der Maßgabe, daß davon mindestens 1 Äquivalent in Form von freier Kalilauge zugesetzt wird.

Durch die überwiegende oder ausschließliche Verwendung von Kaliumverbindungen z.B. Kaliumhydrogensulfit als Komponente B und Kaliumcyanid als Komponente D sowie zusätzliche Zugabe von Kalilauge liegt am Ende der Reaktion im wesentlichen nur gelöstes K₂SO₃ oder eine gelöste Mischung aus K₂SO₃ und Na₂SO₃ vor, so daß sich das ausgefallene Reaktionsprodukt allein durch mechanische Maßnahmen wie Filtration oder Zentrifugieren abtrennen läßt.

Praktisch vollständige Lösung der Salze bedeutet dabei, daß geringe Mengen abgeschiedener Salze nicht stören, da sie nach dem Filtrieren des Produkts ausgewaschen werden können.

Somit führt die Verwendung ausschließlich oder überwiegend von Kaliumverbindungen sowie der Anwendung überschüssiger Alkalilauge zu einer, besonders in größerem Produktionsmaßstab, enormen Verbesserung der Herstellung der α,β-Diaminonitrile.

Dies gilt insbesondere für die Herstellung von 1,4,5,6-Tetrahydro-2-cyanopyrazin, einem wertvollen Zwischenprodukt für Pharmaka, dessen Verwendung in DE-A-44 46 025.2 eingehend erläutert ist.

Um die Umsetzung in Gegenwart von mindestens 3,5 Äquivalenten Alkaliverbindungen, davon mindestens 2,5 Äquivalenten Kaliumverbindungen durchzuführen, besteht die Möglichkeit, entweder als Komponente B von vornherein Alkalihydrogensulfit und als Komponente D Alkalicyanid einzusetzen, sowie zusätzlich mindestens ein Mol Alkalilauge zuzugeben.

Es ist jedoch gleichermaßen möglich, gasförmiges oder flüssiges SO₂ und freie Blausäure zu verwenden und in diesem Fall entsprechende Mengen Alkalilauge (mit überwiegend KOH) zuzugeben, daß insgesamt die Forderung von 3,5 Äquivalenten Alkali, bezogen auf ein Mol der Komponente A, erfüllt ist.

Obgleich die Zugabe der Alkalilauge zu irgendeinem Zeitpunkt der Reaktion erfolgen kann, wird gemäß der bevorzugten Ausführungsform die Reaktion zweckmäßig so durchgeführt, daß bezogen auf ein Mol A mindestens 2 Mol, vorzugsweise 2,2 Mol Alkalihydrogensulfit sowie 1 Mol Alkalicyanid verwendet werden und erst gegen Ende der Reaktion mindestens 1 Mol Alkalihydroxid, vorzugsweise 1,5 Mol Alkalihydroxid, zugegeben wird.

Für die Zugabe von überschüssiger Alkalilauge, d.h. in der Regel freier Kalilauge, besteht nach oben keine Grenze, jedoch wird dadurch kein weiterer Vorteil erzielt, so daß in der Regel nur so viel Kalilauge zugegeben wird, daß die Gesamtmenge von 7 Äquivalenten Kalium im Reaktionsgemisch nicht überstiegen wird.

### Beispiel 1 (Vergleichsbeispiel)

### Verwendung von Natriumsalzen

Man legt 1210 g 38%ige NaHSO₃-Lösung vor, dosiert bei 20 bis 40°C 290 g 40%ige Glyoxallösung zu, gibt bei 20 bis 30°C 120 g Ethylendiamin zu und erwärmt 30 min auf ca. 50°C. Zu der auf 0 bis 5°C gekühlten Lösung läßt man 327 g 30%ige wäßrige NaCN-Lösung zulaufen und rührt noch eine Stunde nach. Anschließend setzt man 240 g 50%ige Natronlauge zu, wobei sich das Reaktionsgemisch auf ca. 45°C erwärmt und sich eine Suspension ausbildet.

Der Niederschlag, bestehend aus Produkt und Na₂SO₃, wird abgesaugt und wieder in Wasser aufgelöst.

Die wäßrige Phase wird dann 20 Stunden kontinuierlich mit Methyltert.-butylether extrahiert. Die resultierende Produkt-Suspension in Methyl-tert.-butylether wird filtriert. Man erhält 122 g Produkt. Ausbeute: 56 %.

### Beispiel 2 (Vergleichsbeispiel)

### Verwendung von Natriumsalzen

Man legt 1210 g 38%ige NaHSO₃-Lösung vor, dosiert bei 20 bis 40°C 290 g 40%ige Glyoxallösung zu, gibt bei 20 bis 30°C 120 g Ethylendiamin zu und erwärmt 30 min auf ca. 50°C. Zu der auf 0 bis 5°C gekühlten Lösung läßt man 327 g 30%ige wäßrige NaCN-Lösung zulaufen und rührt noch eine Stunde nach. Anschließend setzt man 240 g 50%ige Natronlauge zu, wobei sich das Reaktionsgemisch auf ca. 45°C erwärmt. Nach der zweimaligen Extraktion mit je 2000 Volumenteilen Methylethylketon und Eindampfen der vereinigten Extrakte erhält man noch stark verunreinigtes Produkt, welches durch Umkristallisierung aus Methanol gereinigt wird. Es werden 131 g Produkt erhalten. Ausbeute: 60 %.

### Beispiel 3

### Verwendung von Kaliumsalzen

Man legt 1390 g 38%ige KHSO₃-Lösung vor, dosiert bei 20 bis 40°C 290 g 40%ige Glyoxallösung zu, gibt bei 20 bis 30°C 120 g Ethylendiamin zu und erwärmt 30 min auf ca. 50°C. Zu der auf 0 bis 5°C gekühlten Lösung läßt man 434 g 30%ige wäßrige KCN-Lösung zulaufen und rührt noch eine Stunde nach. Anschließend setzt man 336 g 50%ige Kalilauge zu, wobei sich das Reaktionsgemisch auf ca. 45°C erwärmt und sich eine Suspension ausbildet. Der Niederschlag (Produkt) wird abgesaugt und dreimal mit je 150 ml kaltem Wasser gewaschen. Man erhält 135 g Produkt. Ausbeute: 62 %.

## Patentansprüche

1. Verfahren zur Herstellung von α,β-Diaminoacrylnitrilen der allgemeinen Formel I
R³R²NC(R¹)=C (NR²R³)CN I
in welcher
R¹ für Wasserstoff, aliphatische Reste mit 1 bis 30 Kohlenstoffatomen, cycloaliphatische Reste mit 3 bis 8 Ringgliedern oder für aromatische, heteroaromatische oder araliphatische Reste mit 7 bis 12 Kohlenstoffatomen steht, wobei diese Reste auch substituiert sein können;
R² und R³ gleich oder verschieden sind und Wasserstoff oder einen aliphatischen Rest mit 1 bis 15 Kohlenstoffatomen, einen cycloaliphatischen Rest mit 3 bis 8 Ringgliedern oder einen aromatischen, heterocyclischen oder araliphatischen Rest mit 7 bis 12 Kohlenstoffatomen bedeuten und wobei die Reste R² und R³ auch gemeinsam zu einem 5- bis 6-gliedrigen Ring verbunden sein können, der noch ein weiteres Heteroatom tragen kann, oder in der die Reste R² gemeinsam eine Alkylenkette mit 2 bis 3 Kohlenstoffatomen bilden, die noch einen Substituenten R¹ tragen kann, gemäß dem man die Komponente A) mit den Komponenten B), C) und D), das sind
A) eine a-Dicarbonylverbindung der allgemeinen Formel R¹CO-CHO, in der R¹ die o.a. Bedeutung hat oder ein reaktionsfähiges Derivat dieser Carbonylverbindung;
B) ein Salz der schwefligen Säure oder Schwefeldioxid oder ein Schwefeldioxid lieferndes Reagenz;
C) ein Amin der der allgemeinen Formel HNR²R³, oder ein aliphatisches Diamin der allgemeinen Formel R³HN-(CH₂)ₙ-NHR³, in dem die Alkylenkette noch einen Substituenten R¹ tragen kann, wobei R¹ und R³ die o.a. Bedeutung haben und n die Zahlen 2 oder 3 bedeutet, oder Derivate, die unter den Reaktionsbedingungen in das Amin bzw. Diamin übergehend;
D) flüssige oder gasförmige Blausäure oder ein Blausäure lieferndes Reagenz in beliebiger Reihenfolge umsetzt,
dadurch gekennzeichnet, daß man die Umsetzung, bezogen auf ein Mol der Komponente A, mit mindestens 3,5 Äquivalenten Alkaliverbindungen durchführt, wovon der überwiegende Teil aus Kaliumverbindungen besteht und wobei mindestens ein Äquivalent Alkali in Form von freier Alkalilauge zugesetzt wird, mit der Maßgabe, daß man den Anteil an Kaliumäquivalenten mindestens so hoch wählt, daß am Ende der Reaktion alle anorganische Salze praktisch vollständig in Lösung bleiben.

2. Verfahren gemäß Anspruch 1, dadurch gekennzeichnet, daß man die Umsetzung, bezogen auf ein Mol der Komponente A, mit mindestens 3,5 Äquivalenten Alkaliverbindungen durchführt, wovon mindestens 2,5 Äquivalente Kalium sind und mit der Maßgabe, daß mindestens 1 Äquivalent Alkali in Form von freier Alkalilauge zugesetzt wird.

3. Verfahren gemäß Anspruch 1, dadurch gekennzeichent, daß man die Umsetzung ausschließlich mit Kaliumverbindungen durchführt, wobei die Gesamtmenge des Kaliums, bezogen auf die Komponente A, mindestens 4 Äquivalente beträgt, mit der Maßgabe, daß davon mindestens 1 Äquivalent in Form von freier Kalilauge zugesetzt wird.

4. Verfahren gemäß Anspruch 1, dadurch gekennzeichnet, daß man als Komponente B Kaliumhydrogensulfit und als Komponente D Kaliumcyanid verwendet.

5. Verfahren gemäß Anspruch 4, dadurch gekennzeichnet, daß mindestens 1 Mol Kalilauge, bezogen auf A, gegen Ende der Reaktion zugegeben wird.

6. Verfahren gemäß Anspruch 1, dadurch gekennzeichnet, daß man als Komponente A Glyoxal und als Komponente C Ethylendiamin zur Herstellung von 1,4,5,6-Tetrahydro-2-cyanopyrazin verwendet.

## Claims

1. A process for preparing α,β-diaminoacrylonitriles of the formula I
R³R²NC(R¹)=C(NR²R³)CN I
where
R¹ is hydrogen, aliphatic radicals having from 1 to 30 carbon atoms, cycloaliphatic radicals having 3 to 8 ring members or aromatic, heteroaromatic or araliphatic radicals having 7 to 12 carbon atoms, it also being possible for these radicals to be substituted;
R² and R³ are identical or different and are hydrogen or an aliphatic radical having 1 to 15 carbon atoms, a cycloaliphatic radical having 3 to 8 ring members or an aromatic, heterocyclic or araliphatic radical having 7 to 12 carbon atoms, and where the radicals R² and R³ can also be connected together to give a 5- to 6-membered ring which may also carry another hetero atom, or in which the R² radicals together form an alkylene chain which has 2 to 3 carbon atoms and may also carry a substituent R¹, in which there is reaction in any sequence of component A) with components B), C) and D), which are
A) an α-dicarbonyl compound of the formula R¹COCHO where R¹ has the abovementioned meaning, or a reactive derivative of this carbonyl compound;
B) a salt of sulfurous acid or sulfur dioxide or a reagent providing sulfur dioxide;
C) an amine of the formula HNR²R³ or an aliphatic diamine of the formula R³HN-(CH₂)ₙ-NHR³ in which the alkylene chain may also carry a substituent R¹, where R¹ and R³ have the abovementioned meaning, and n is the numbers 2 or 3, or derivatives which are converted under the reaction conditions into the amine or diamine;
D) liquid or gaseous hydrocyanic acid or a reagent providing hydrocyanic acid,
wherein the reaction is carried out with at least 3.5 equivalents of alkali metal compounds, most of which consist of potassium compounds, per mole of component A, and where at least one equivalent of alkali metal is added in the form of free alkali metal hydroxide solution, with the proviso that the proportion of potassium equivalents is chosen to be at least high enough for all the inorganic salts to remain virtually completely in solution at the end of the reaction.

2. A process as claimed in claim 1, wherein the reaction is carried out with at least 3.5 equivalents of alkali metal compounds, of which at least 2.5 equivalents are potassium, per mole of component A, and with the proviso that at least 1 equivalent of alkali metal is added in the form of free alkali metal hydroxide solution.

3. A process as claimed in claim 1, wherein the reaction is carried out exclusively with potassium compounds, with the total amount of potassium being at least 4 equivalents based on component A, with the proviso that at least 1 equivalent thereof is added in the form of free potassium hydroxide solution.

4. A process as claimed in claim 1, wherein potassium bisulfite is used as component B and potassium cyanide is used as component D.

5. A process as claimed in claim 4, wherein at least 1 mol of potassium hydroxide solution based on A is added toward the end of the reaction.

6. A process as claimed in claim 1, wherein glyoxal is used as component A and ethylenediamine is used as component C for preparing 1,4,5,6-tetrahydro-2-cyanopyrazine.

## Revendications

1. Procédé pour la préparation d'α,β-diaminoacrylonitriles de formule générale I
R³R²NC(R¹)=C (NR²R³) CN I
dans laquelle
R¹ représente un atome d'hydrogène ou un radical aliphatique ayant de 1 à 30 atomes de carbone, un radical cycloaliphatique ayant de 3 à 8 chaînons formant le cycle ou un radical aromatique, hétéroaromatique ou araliphatique ayant de 7 à 12 atomes de carbone, ces radicaux pouvant également être substitués;
R² et R³ sont identiques ou différents et représentent un atome d'hydrogène ou un radical aliphatique ayant de 1 à 15 atomes de carbone, un radical cycloaliphatique ayant de 3 à 8 chaînons formant le cycle ou un radical aromatique, hétérocyclique ou araliphatique ayant de 7 à 12 atomes de carbone, et les radicaux R² et R³ pouvant également être liés l'un à l'autre en un cycle à 5 ou 6 chaînons, qui peut encore porter un autre hétéro-atome, ou dans laquelle les radicaux R² forment ensemble une chaîne alkylène ayant 2 ou 3 atomes de carbone, qui peut encore porter un substituant R¹,
selon lequel on fait réagir le composant A) avec, en un ordre quelconque, les composants B), C) et D), qui sont
A) un composé α-dicarbonyle de formule générale R¹COCHO, dans laquelle R¹ a la signification donnée ci-dessus, ou un dérivé réactif de ce composé carbonyle;
B) un sel de l'acide sulfureux ou l'anhydride sulfureux ou un réactif fournissant de l'anhydride sulfureux;
C) une amine de formule générale HNR²R³ ou une diamine aliphatique de formule générale R³HN-(CH₂)ₙ-NHR₃, dans laquelle la chaîne alkylène peut encore porter un substituant R¹, R¹ et R³ ayant les significations données ci-dessus et n représentant le nombre 2 ou 3, ou des dérivés qui sont convertis en l'amine ou la diamine dans les conditions réactionnelles;
D) l'acide cyanhydrique liquide ou gazeux ou un réactif fournissant de l'acide cyanhydrique,
caractérisé en ce que l'on effectue la réaction avec, par rapport à 1 mole du composant A, au moins 3,5 équivalents de composés alcalins, la majeure partie de ceux-ci consistant en composés à base de potassium et au moins 1 équivalent de composé alcalin étant ajouté sous forme de solution alcaline libre, étant entendu que l'on choisit la quantité d'équivalents potassiques au moins suffisante pour qu'à la fin de la réaction tous les sels minéraux restent pratiquement totalement en solution.

2. Procédé selon la revendication 1, caractérisé en ce que l'on effectue la réaction avec, par rapport à 1 mole du composant A, au moins 3,5 équivalents de composés alcalins, dont au moins 2,5 équivalents consistent en potassium, et étant entendu qu'au moins 1 équivalent de composé alcalin est ajouté sous forme de solution alcaline libre.

3. Procédé selon la revendication 1, caractérisé en ce que l'on effectue la réaction exclusivement avec des composés à base de potassium, la quantité totale du potassium, par rapport au composant A, étant d'au moins 4 équivalents, étant entendu que parmi ceux-ci, au moins 1 équivalent est ajouté sous forme de solution d'hydroxyde de potassium libre.

4. Procédé selon la revendication 1, caractérisé en ce que l'on utilise comme composant B de l'hydrogéno-sulfite de potassium et, en tant que composant D, du cyanure de potassium.

5. Procédé selon la revendication 4, caractérisé en ce que, vers la fin de la réaction, on ajoute au moins 1 mole de solution d'hydroxyde de potassium, par rapport à A.

6. Procédé selon la revendication 1, caractérisé en ce que, pour la préparation de la 1,4,5,6-tétrahydro-2-cyanopyrazine, on utilise comme composant A du glyoxal et, comme composant C, de l'éthylènediamine.
